# EUROPEAN PATENT SPECIFICATION

(11) **EP 2 167 025 B1**
(45) Date of publication and mention of the grant of the patent: **08.08.2018**
(21) Application number: 08757266.5
(22) Date of filing: 17.06.2008
(51) Int. Cl.: A61K 8/33, A61K 8/34, A61K 8/368, A61Q 17/00, A01N 31/08

(54) **PRESERVATIVE-FREE COMPOSITIONS COMPRISING CINNAMIC OR ANISIC ACID AND A BENZALDEHYDE (DERIVATIVE)**
KONSERVIERUNGSMITTELFREIE ZUSAMMENSETZUNGEN MIT ZIMT- ODER ANISSÄURE UND EINEM BENZALDEHYD (DERIVAT)
COMPOSITIONS SANS CONSERVATEURS COMPRENANT L'ACIDE CINNAMIQUE OU ANISIQUE ET UN (DÉRIVÉ DE) BENZALDÉHYDE

(30) Priority: 22.06.2007 GB 0712024
(43) Date of publication of application: 31.03.2010
(73) Proprietor: Givaudan SA, 1214 Vernier (CH)
(72) Inventor: NATSCH, Andreas, CH-8707 Uetikon (CH)
(74) Representative: Simmons, John Murray
(86) International application number: PCT/CH2008/000273
(87) International publication number: WO 2009/000097

(56) References cited:
- EP-A- 0 618 285
- DE-U1-202006 001 274
- GB-A- 1 447 653
- JP-A- 2004 083 468
- NL-C1- 1 026 972
- US-A- 5 214 027
- US-A- 5 852 060
- US-A1- 2001 006 666
- US-A1- 2001 014 315
- US-A1- 2002 155 086
- US-A1- 2005 025 846
- LEUNG A Y; FOSTER S: "Encyclopedia of Common Natural Ingredients used in food, drugs and cosmetics" 1996, WILEY , NEW YORK , XP002524740 Pages 36-37, 81, 167
- ARSLAN NESET; GURBUZ BILAL; SARIHAN ERCUMENT O; BAYRAK ALI; GUMUSCU AHMET: "Variation in essential oil content and composition in Turkish Anise (Pimpinella anisum L.) Populations", TURKISH JOURNAL OF AGRICULTURE AND FORESTRY, vol. 28, no. 3, 2004, page 173-177,
- MODUGNO ET AL: "Aromatic resin characterisation by gas chromatography-mass spectrometry", JOURNAL OF CHROMATOGRAPHY, ELSEVIER SCIENCE PUBLISHERS B.V, NL, vol. 1134, no. 1-2, 17 November 2006 (2006-11-17), pages 298-304, XP005719705, ISSN: 0021-9673, DOI: 10.1016/J.CHROMA.2006.09.010
- WU ET AL: "Secondary metabolites from the roots of Engelhardia roxburghiana and their antitubercular activities", PHYTOCHEMISTRY, PERGAMON PRESS, GB, vol. 68, no. 9, 21 April 2007 (2007-04-21) , pages 1338-1343, XP022041647, ISSN: 0031-9422, DOI: 10.1016/J.PHYTOCHEM.2007.01.018
- ELZAAWELY ET AL: "Antioxidant activity and contents of essential oil and phenolic compounds in flowers and seeds of Alpinia zerumbet (Pers.) B.L. Burtt. & R.M. Sm", FOOD CHEMISTRY, ELSEVIER LTD, NL, vol. 104, no. 4, 1 January 2007 (2007-01-01), pages 1648-1653, XP022095496, ISSN: 0308-8146, DOI: 10.1016/J.FOODCHEM.2007.03.016
- CHANG S T ET AL: "Antibacterial activity of leaf essential oils and their constituents from Cinnamomum osmophloeum", JOURNAL OF ETHNOPHARMACOLOGY, ELSEVIER IRELAND LTD, IE, vol. 77, no. 1, 1 September 2001 (2001-09-01), pages 123-127, XP027380307, ISSN: 0378-8741 [retrieved on 2001-09-01]

## Description

Provided are essentially preservative-free personal care product compositions and their use in personal care products applied to the human skin or scalp, and methods of making such products.

Preservatives are used in personal care products (products applied to the skin or scalp either to remain there or to be rinsed off) to preserve these products against microbial spoilage and to extend their shelf life.

Antimicrobial compounds used for product preservation may fall into one or more of the following classes based on the effect they have on the microorganism, in particular bacteria and fungi. An antibacterial or antifungal may inhibit growth of the microorganisms or kill them or both. A bacteriostatic compound inhibits growth of bacteria, while a bactericide kills bacteria (reduces their number). Similarly, a fungistatic compound inhibits the growth of fungi (molds and yeast), while a fungicide kills fungi (reduces their number). A sporicide kills spores of fungi or bacteria. Spores, especially endospores, are formed by some bacteria to survive during periods of deprivation and are significantly more difficult to kill. Fungi form spores for reproduction and these spores are significantly more difficult to kill than the vegetative form of the fungi. Many antimicrobial compounds are therefore not effective against fungal spores.

A broad band preservative effect including a bactericidal and fungicidal activity was previously only attained in personal care products by addition of certain preservatives, in particular formaldehyde, formaldehyde donors, halogenated compounds, compounds belonging to the class of parabens and a variety of specific fungicides.

Formaldehyde donors include in particular diazolidinyl urea (CAS 78491-02-8), imidazolidinyl urea (CAS 39236-46-9), and DMDM Hydantoin (CAS 6440-58-0).

Halogenated compounds include in particular 2,4-dichlorobenzyl-alcohol (CAS 1777-82-8), Chloroxylenol (also known as 4-chloro-3,5-dimethyl-phenol, CAS 88-04-0), Bronopol (also known as 2-bromo-2-nitropropane-1,3-diol, CAS 52-51-7), iodopropynyl butyl carbamate (CAS 55406-53-6).

Paraben compounds include in particular Methyl-paraben (CAS 99-76-3), Ethyl-paraben (CAS 120-47-8), Propyl-paraben (CAS 94-13-3), Butyl-paraben (CAS 94-26-8), Isopropyl-paraben (CAS 4191-73-5), and Benzyl-paraben (CAS 94-18-8).

Other preservatives include Quaternium-15 (CAS 51229-78-8), methyl-chloroisothiazolinone (CAS 26172-55-4), and methylisothiazolinone (CAS 2682-20-4).

There are concerns that some of these preservatives may constitute health hazards, for example, iodopropynyl butyl carbamate, formaldehyde and formaldehyde donors, methyl-chloroisothiazolinone (CAS 26172-55-4), and methylisothiazolinone are considered highly allergenic/sensitizing.

Accordingly there is an interest in removing preservatives from personal care products applied to human skin or scalp while maintaining a good broad band stabilisation of the product against microbial spoilage including a sporicidal effect. Whilst D4 (US2005/0025846A1) discloses that certain essential oils, including anise oil, may act as preservatives in formulations comprising biologically active macromolecules (see paragraphs [0023-0024]), D4 only discloses that compositions may contain anise oil in a content of 0.1% to 20% (v/v) with no mention of the individual components of anisic oil.

Applicant suprisingly identified a combination of fragrance compounds that enables to provide essentially preservative-free personal care product compositions that display a stabilizing effect, in particular a broadband antimicrobial action including a sporicidal effect. Due to their well known characteristics and their primary use as fragrance, the compounds of the discovered combination are not considered preservatives in the industry and accordingly do not have to be labelled as such.

The stabilizing effect is reached by the following combination of fragrance compounds:
a) benzaldehyde and benzaldehyde-derivatives according to formula I as defined herein below and
b) fragrant acids selected from the group consisting of cinnamic acid and anisic acid according to formula II as defined herein-below.

Some of these benzaldehyde and benzaldehyde derivative compounds have previously been shown to have a fungistatic effect against various food spoilage molds and yeasts. The antifungal activity of a given antifungal against a given fungal species varies with the food product in which it is used, possibly due to the concentration of lipids or proteins. Fitzgerald et al. report vanillin and various derivatives to have antifungal (fungistatic) activity against a variety of food molds including various Aspergillus species (A. oryzae, A sojae), Penicillium species, and yeast strains when tested in yeast extract peptone dextrose broth. The efficacy against various fungal strains varies. Fungicidal or sporicidal activities were not tested. (J. Agric. Food Chem. 2005, 53, 1769-1775).

Similarly, heliotropin is known to be active as a fungistatic compound in vaporous form when applied to fungi on tobacco leaves, and to have an antifungal and antibacterial effect against some fungi and bacteria in aqueous culture media.

While many substituted benzaldehydes and benzylalcohols are known to have a germistatic activity against some microorganisms, the germicidal effect, in particular the bactericidal and fungicidal effect, is generally considered to be low, especially when the pH is within the range commonly used in personal care products which is pH 5 to pH 9. While some compounds are known to be more active under extremely acidic or alkaline conditions, this effect does not extend to the pH range used in personal care products.

That compounds that are fungistatic in certain food stuffs can provide a fungicidal and sporicidal effect in personal care products that often contain lipids and proteins or a high concentration of detergents was completely surprising and could not have been predicted. An activity or lack of activity of a given test compound in water is not indicative of an activity in a personal care product, for example a cosmetic cream.

### SUMMARY

In a first aspect, there is provided:
1. A personal care product composition comprising
   a) at least one benzaldehyde or benzaldehyde-derivative compound of formula I, 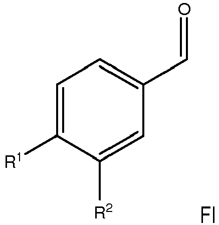
      wherein R¹ is a residue selected from the group consisting of H, CH₂, CH₃, OH, and OCH₃,
      wherein R² is a residue selected from the group consisting of H, OH, OCH₃, and OCH₂O, and
      wherein if R² is OCH₂O, then R¹ is CH₂ and forms a bond with R², selected from the group consisting of Benzaldehyde, 4-methylbenzaldehyde, Heliotropine, Vanillin, 4-hydroxybenzaldehyde, 3-hydroxybenzaldehyde, 4-methoxybenzaldehyde, 3-methoxybenzaldehyde, and 3-hydroxy-4-methoxybenzaldehyde (isovanillin); and
   b) at least one fragrant acid according to formula II

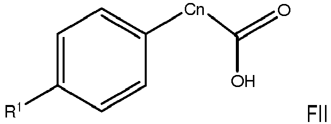

   wherein R¹ is a residue selected from the group consisting of H and OCH₃,
   wherein n is selected from 0 or 2, and
   wherein if R¹ is H then n is 2, and the C atoms of the resulting C₂ situated between aromatic ring and COOH form a double bond, and
   wherein if R¹ is OCH₃ then n is 0;
   selected from the group consisting of cinnamic acid and anisic acid;
   wherein the at least one benzaldehyde or benzaldehyde-derivative compound a) is present in a total concentration of 0.05 to 0.5% (w/w);
   wherein the at least one fragrant acid b) is present in a total concentration of 0.05 to 0.5% (w/w); and a cosmetically-acceptable base,
   with the proviso that the personal care product composition is essentially free of a preservative selected from the group consisting of Formaldehyde paraformaldehyde, Biphenyl-2-ol(o-phenylphenol) and its salts, Pyrithione zinc, Inorganic sulphites and hydrogen- sulphites, Sodium iodate, Chlorobutanol, 4-Hydroxybenzoic acid and its salts and esters, 3-Acetyl-6-methylpyran-2,4 (3H)-dione (Dehydracetic acid) and its salts, Formic acid and its sodium salt, 3,3'-Dibromo-4,4'-hexamethylenedioxydibenzamidine (Dibromohexamidine) and its salts (including isethionate), Thiomersal, Phenylmercuric salts (including borate), Hexetidine, 5-Bromo-5-nitro-1,3-dioxane, Bronopol, 2,4-Dichlorobenzyl alcohol, Triclocarban, 4-Chloro-m-cresol, 4-Chloro-3,5-xylenol, 3,3'-Bis(1-hydroxymethyl-2,5-dioxoimidazolidin-4-yl)-1,1'-methylenediurea ('Imidazolidinyl urea'), Hexamethylenetetramine (methenamine), Methenamine 3-chloroallylochloride, 1-(4-Chlorophenoxy)-1-(imidazol-1-yl)-3,3-dimethylbutan-2-one, 1,3-Bis(hydroxymethyl)-5,5-dimethylimidazolidine-2,4-dione, 1-Hydroxy-4-methyl-6(2,4,4-trimethylpentyl) 2-pyridon and its monoethanolamine salt, 1,2-Dibromo-2,4-dicyanobutane (methyldibromoglutaronitrile), 6,6-Dibromo-4,4-dichloro2,2'-methylenediphenol (Bromochlorophen), Mixture of 5-Chloro-2-methyl-isothiazol-3 (2H)-one and 2-methylisothiazol-3(2H)-one with magnesium chloride and magnesium nitrate, 2-Benzyl-4-chlorophenol (chlorophene), 2-Chloroacetamide, Chlorhexidine and its digluconate, diacetate and dihydrochloride, Alkyl (C12-C22) trimethyl ammonium, bromide and chloride, 4,4-dimethyl-1,3-oxizalidine, N-(Hydroxymethyl)-N-(dihydroxymethyl-1,3-dioxo-2,5-imidazolidinyl-4)-N'-(hydroxymethyl) urea, 1,6-Di(4-amidinophenoxy)-n-hexane (Hexamidine) and its salts (including isethionate and p-hydroxybenzoate), Glutaraldehyde (Pentane-1,5-dial), 5-Ethyl-3,7-dioxa-1-azabicyclo[3.3.0]octane, 3-(p-chlorophenoxy)-propane-1,2 diol (chlorphenesin), Silver chloride deposited on titanium dioxide, Benzethonium Chloride, Benzalkonium chloride, bromide and saccharinate, Benzylhemiformal , 3-Iodo-2-propynylbutylcarbamate , and Methylisothiazolinone.
   characterized in that the composition comprises:
   as component (a) 4-hydroxybenzaldehyde; and
   as component (b) anisic acid.
2. The personal care product composition according to paragraph 1 wherein the at least one benzaldehyde or benzaldehyde-derivative compound a) is present in a concentration of 0.075 to 0.3% (w/w), optionally 0.1 to 0.2%, (w/w), and wherein the at least one fragrant acid b) is present in a concentration of 0.075 to 0.3% (w/w), optionally 0.1 to 0.2% (w/w).
3. The personal care product composition according to any one of paragraphs 1 and 2 further comprising at least one aromatic alcohol or derivative thereof selected from the group consisting of phenylethyl alcohol, phenyl propyl alcohol, benzyl formiate, and phenethyl formiate.
4. The personal care product composition according to any one of paragraphs 1 and 2 further comprising an antimicrobial fragrance ingredient selected from the group consisting of Geranium oil, Peppermint oil, Rose oil, Cinnamon leaf oil, Fucus oil, Clove bud oil, Clove leaf oil, Palmarosa oil, Citrus oil, Terpene fraction of citrus oil, Orange oil, Terpene fraction of orange oil, Geraniol, Cuminic alcohol, Perilla alcohol, Citronellol, Eugenol, Cinnamic alcohol, Nerol, Menthol, Borneol, Octan-1-ol, Nonan-1-ol, Decan-1-ol, Dec-9-en-1-ol, Limonene, 2,6-dimethyl-7-octen-2-ol, 3-methyl-5-phenyl-pentanol, 2-methyl-5-phenyl-pentanol, undec-10-en-1-ol, 4-(1-methylethyl)-cyclohexanol, 4-(1,1-dimethylethyl)-cyclohexanol, 2,2-dimethyl-3-(3-methyl phenyl)-propanol, 4-(1-methylethyl)cyclohexyl-methanol, (E)-2-(3,3-dimethylbicyclo[2.2.1]hept-2-ylidene)-ethanol, 3,7-dimethyl-7-octen-1-ol, 2-methoxy-4-propyl-phenol, 3-(4-methyl-3-cyclohexenyl)-butanol, Dihydroterpineol, 2,6-dimethylheptan-2-ol, 3-(4-methoxyphenyl)-2-methyl- Propanal, Octahydro-5-methoxy- 4,7-Methano-1H-indene-2-carboxaldehyde, alpha-methyl- 1,3-Benzodioxole-5-propanal, 3-[4-(1,1-dimethylethyl)phenyl]propanal, 8-(1-methylethyl)-1-oxaspiro[4.5]-decan-2-one, Tetrahydro-6-(2Z)-2-pentenyl-2H-Pyran-2-one, 5-(3Z)-3-hexenyldihydro-2(3H)-Furanone, 5-hexyldihydro-5-methyl-2(3H)-Furanone, 4-methyl-5-pentyl-dihydo-2(3H)-Furan-2-one, 6-hexyltetrahydro-2H-pyran-2-one, 5-hexyl-Furan-2(3H)-one, 6-heptyltetrahydro-2H-Pyran-2-one, 5-octyl-furan-2-one, Dihydro-5-pentyl-2(3H)-Furanone, 5-heptyldihydro-(2(3H)-Furanone), and 6-hexyltetrahydro-2H-Pyran-2-one.
5. The personal care product composition according to any one of paragraphs 1 and 2 further comprising at least one aromatic alcohol or derivative thereof as defined in paragraph 3 and at least one antimicrobial fragrance ingredient as defined in paragraph 4.
6. A composition according to any one of paragraphs 1 to 5 selected from compositions for personal care products applied to and left on the skin or scalp including but not limited to creams, salves, lotions, and ointments for hand, face or body, perfumes, eau de Cologne, eau de toilet, deodorants, antiperspirants, and products applied but rinsed off including but not limited to soaps, liquid soaps, shower gels, and shampoos.
7. Use of at least one benzaldehyde or benzaldehyde-derivative compound a) as defined in any one of the claims 1 to 6 and at least one fragrant acid b) as defined in any one of the paragraphs 1 to 6 for the preparation of a preserved personal care product composition as defined in any one of paragraphs 1-6.
8. Method of forming a stabilized personal care product composition as defined in any one of paragraphs 1-6 which is sufficiently bactericidal to have a reduction factor for Pseudomonas aeruginosa and Staphylococcus aureus of at least 1000 per 7 days, and which is sufficiently sporicidal to have a reduction factor of at least 100 per 7 days for Aspergillus niger, by admixing an effective amount of at least one benzaldehyde or benzaldehyde-derivative compound a) and an effective amount of at least one fragrant acid b) as defined in any one of paragraphs 1 to 6 to a personal care product base, wherein optionally said compounds a) and b) may be added in form of a pre-prepared fragrance composition comprising said compounds a) and b), forming a personal care product composition.
9. Method according to paragraph 8 wherein the total concentration of the at least one benzaldehyde or benzaldehyde-derivative compound a) in the personal care product composition is from 0.3 to 0.5%, and the total concentration of the at least one fragrant acid b) in the personal care product composition is from 0.3 to 0.5%.
10. Method of any one of paragraphs 8 and 9 wherein the personal care product composition formed further comprises at least one aromatic alcohol or derivative thereof selected from the group consisting of phenylethyl alcohol, phenyl propyl alcohol, benzyl formiate, and phenethyl formiate.
11. Method of any one of paragraphs 8 and 9 wherein the personal care product composition formed further comprises at least one antimicrobial fragrance ingredient as defined in paragraph 4.
12. Method of any one of paragraphs 8 and 9 wherein the personal care product composition formed further comprises at least one aromatic alcohol or derivative thereof selected from the group consisting of phenylethyl alcohol, phenyl propyl alcohol, benzyl formiate, and phenethyl formiate; and at least one antimicrobial fragrance ingredient as defined in paragraph 4.
13. Method of any one of paragraph 8 and 9 wherein the personal care product composition is or is brought into the form of a personal care product including sticks, roll-ons, sprays, pump-sprays, aerosols, soap bars, powders, solutions, gels, creams, balms and lotions.

In another embodiment, there is provided a personal care product composition as described herein, wherein the at least one benzaldehyde or benzaldehyde-derivative compound a) is present in a concentration of 0.075 to 0.3% (w/w), optionally 0.1 to 0.2%, (w/w), and wherein the at least one fragrant acid b) is present in a concentration of 0.075 to 0.3% (w/w), optionally 0.1 to 0.2% (w/w).

In another embodiment, there is provided a personal care product composition as described herein, further comprising at least one aromatic alcohol or derivative thereof selected from the group consisting of phenylethyl alcohol, phenyl propyl alcohol, benzyl formiate, and phenethyl formiate.

In another embodiment, there is provided a personal care product composition as described herein, further comprising an antimicrobial fragrance ingredient selected from the group consisting of Geranium oil, Peppermint oil, Rose oil, Cinnamon leaf oil, Fucus oil, Clove bud oil, Clove leaf oil, Palmarosa oil, Citrus oil, Terpene fraction of citrus oil, Orange oil, Terpene fraction of orange oil, Geraniol, Cuminic alcohol, Perilla alcohol, Citronellol, Eugenol, Cinnamic alcohol, Nerol, Menthol, Borneol, Octan-1-ol, Nonan-1-ol, Decan-1-ol, Dec-9-en-1-ol, Limonene, 2,6-dimethyl-7-octen-2-ol, 3-methyl-5-phenyl-pentanol, 2-methyl-5-phenyl-pentanol, undec-10-en-1-ol, 4-(1-methylethyl)-cyclohexanol, 4-(1,1-dimethylethyl)-cyclohexanol, 2,2-dimethyl-3-(3-methyl phenyl)-propanol, 4-(1-methylethyl)cyclohexyl-methanol, (E)-2-(3,3-dimethylbicyclo[2.2.1]hept-2-ylidene)-ethanol, 3,7-dimethyl-7-octen-1-ol, 2-methoxy-4-propyl-phenol, 3-(4-methyl-3-cyclohexenyl)-butanol, Dihydroterpineol, 2,6-dimethylheptan-2-ol, 3-(4-methoxyphenyl)-2-methyl- Propanal, Octahydro-5-methoxy-4,7-Methano-1H-indene-2-carboxaldehyde, alpha-methyl- 1,3-Benzodioxole-5-propanal, 3-[4-(1,1-dimethylethyl)phenyl]propanal, 8-(1-methylethyl)-1-oxaspiro[4.5]-decan-2-one, Tetrahydro-6-(2Z)-2-pentenyl-2H-Pyran-2-one, 5-(3Z)-3-hexenyldihydro-2(3H)-Furanone, 5-hexyldihydro-5-methyl- 2(3H)-Furanone, 4-methyl-5-pentyl-dihydo-2(3H)-Furan-2-one, 6-hexyltetrahydro-2H-pyran-2-one, 5-hexyl-Furan-2(3H)-one, 6-heptyltetrahydro-2H-Pyran-2-one, 5-octyl -furan-2-one, Dihydro-5-pentyl-2(3H)-Furanone, 5-heptyldihydro-(2(3H)-Furanone), and 6-hexyltetrahydro- 2H-Pyran-2-one.

In another embodiment, there is provided a personal care product composition as described herein, further comprising at least one aromatic alcohol or derivative thereof as described herein, and at least one antimicrobial fragrance ingredient as described herein.

In another embodiment, there is provided a composition as described herein, selected from compositions for personal care products applied to and left on the skin or scalp including but not limited to creams, salves, lotions, and ointments for hand, face or body, perfumes, eau de Cologne, eau de toilet, deodorants, antiperspirants, and products applied but rinsed off including but not limited to soaps, liquid soaps, shower gels, and shampoos.

In another embodiment, there is provided a personal care product comprising the composition as described herein, selected from an application form selected from stick, roll-on, spray, pump-spray, aerosol, soap bar, powder, solution, gel, cream, balm and lotion.

In another embodiment, there is provided a personal care product or composition therefor as described herein, wherein the personal care product composition comprises lipids.

In another embodiment, there is provided a personal care product or composition therefor as described herein, wherein the composition is an emulsion.

In another embodiment, there is provided a personal care product or a personal care product composition as described herein, wherein the pH is 5 to 9.

In another embodiment, there is provided a personal care product composition as described herein, wherein the benzaldehyde or benzaldehyde-derivative compound a) and the fragrant acid b) as described herein, the aromatic alcohols or derivatives thereof as described herein, and the antimicrobial fragrance ingredients as described herein that are present in the composition exclusively are compounds or ingredients that occur in nature.

In another embodiment, there is provided a personal care product composition as described herein, wherein all ingredients of the composition consists exclusively of compounds or ingredients that occur in nature.

In another embodiment, there is provided a personal care product composition as described herein, wherein the compounds or ingredients that occur in nature have been extracted or purified from a natural source including but not limited to a botanical source, or that have been formed by natural processes including but not limited to fermentation.

In another embodiment, there is provided the use of at least one benzaldehyde or benzaldehyde-derivative compound a) as described herein, and at least one fragrant acid b) as described herein, for the preparation of a preserved personal care product composition, or a preserved personal care product.

In another embodiment, there is provided a method of forming a stabilized personal care product which is sufficiently bactericidal to have a reduction factor for Pseudomonas aeruginosa and Staphylococcus aureus of at least 1000 per 7 days, and which is sufficiently sporicidal to have a reduction factor of at least 100 per 7 days for Aspergillus niger.
by admixing an effective amount of at least one benzaldehyde or benzaldehyde-derivative compound a) and an effective amount of at least one fragrant acid b) as described herein, to a personal care product base, wherein optionally said compounds a) and b) may be added in form of a pre-prepared fragrance composition comprising said compounds a) and b), forming a personal care product composition as described herein.

In another embodiment, there is provided a method as described herein, wherein the total concentration of the at least one benzaldehyde or benzaldehyde-derivative compound a) in the personal care product composition is from 0.05% to 1 % (w/w), optionally from 0.3 to 0.7%, and the total concentration of the at least one fragrant acid b) in the personal care product composition is from 0.05% to 1 % (w/w), optionally 0.3 to 0.7%.

In another embodiment, there is provided a method as described herein wherein the personal care product composition formed further comprises at least one aromatic alcohol or derivative thereof selected from the group consisting of phenylethyl alcohol, phenyl propyl alcohol, benzyl formiate, and phenethyl formiate.

In another embodiment, there is provided a method as described herein, wherein wherein the personal care product composition formed further comprises at least one antimicrobial fragrance ingredient as described herein.

In another embodiment, there is provided a method as described herein, wherein the personal care product composition formed further comprises at least one aromatic alcohol or derivative thereof selected from the group consisting of phenylethyl alcohol, phenyl propyl alcohol, benzyl formiate, and phenethyl formiate; and at least one antimicrobial fragrance ingredient as described herein.

In another embodiment, there is provided a method as described herein, wherein the personal care product composition is or is brought into the form of a personal care product including sticks, roll-ons, sprays, pump-sprays, aerosols, soap bars, powders, solutions, gels, creams, balms and lotions.

### DETAILED DESCRIPTION

The personal care products described herein are essentially preservative-free, meaning that they may contain only traces of preservatives as herein defined, whose concentration is substantially below their effective antimicrobial concentration, for example, at least 5 times or 10 times less. Such traces can be due to, for example, impurities of one or more ingredients of a personal care product or preserved ingredients/excipients used in low concentrations in personal care products.

The term "preservative" as used herein refers to a preservative or combination of preservatives listed herein below. Preservatives are substances which may be added to personal care products for the primary purpose of inhibiting the development of micro-organisms in such products. 'Salts' of preservatives is taken to mean: salts of the cations sodium, potassium, calcium, magnesium, ammonium and ethanolamines; salts of the anions chloride, bromide, sulphate, acetate. 'Esters' of preservatives is taken to mean: esters of methyl, ethyl, propyl, isopropyl, butyl, isobutyl, phenyl.

### Preservatives:

Formaldehyde paraformaldehyde
Biphenyl-2-ol (o-phenylphenol) and its salts
Pyrithione zinc (INN)
Inorganic sulphites and hydrogen- sulphites
Sodium iodate
Chlorobutanol (INN)
4-Hydroxybenzoic acid and its salts and esters
3-Acetyl-6-methylpyran-2,4 (3H)-dione (Dehydracetic acid) and its salts
Formic acid and its sodium salt
3,3'-Dibromo-4,4'-hexamethylenedioxydibenzamidine (Dibromohexamidine) and its salts (including isethionate)
Thiomersal (INN)
Phenylmercuric salts (including borate)
Hexetidine (INN)
5-Bromo-5-nitro-1,3-dioxane
Bronopol (INN)
2,4-Dichlorobenzyl alcohol
Triclocarban (INN)
4-Chloro-m-cresol
4-Chloro-3,5-xylenol
3,3'-Bis(1-hydroxymethyl-2,5-dioxoimidazolidin-4-yl)-1,1'-methylenediurea ('Imidazolidinyl urea') Hexamethylenetetramine (methenamine) (INN)
Methenamine 3-chloroallylochloride (INNM)
1-(4-Chlorophenoxy)-1-(imidazol-1-yl)-3,3-dimethylbutan-2-one
1,3-Bis(hydroxymethyl)-5,5-dimethylimidazolidine-2,4-dione
1-Hydroxy-4-methyl-6(2,4,4-trimethylpentyl) 2-pyridon and its monoethanolamine salt 1,2-Dibromo-2,4-dicyanobutane (methyldibromoglutaronitrile)
6,6-Dibromo-4,4-dichloro2,2'-methylenediphenol (Bromochlorophen)
Mixture of 5-Chloro-2-methyl-isothiazol-3 (2H)-one and 2-methylisothiazol-3(2H)-one with magnesium chloride and magnesium nitrate
2-Benzyl-4-chlorophenol (chlorophene)
2-Chloroacetamide
Chlorhexidine (INN) and its digluconate, diacetate and dihydrochloride
Alkyl (C12-C22) trimethyl ammonium, bromide and chloride
4,4-dimethyl-1,3-oxizalidine
N-(Hydroxymethyl)-N-(dihydroxymethyl-1,3-dioxo-2,5-imidazolidinyl-4)-N'-(hydroxymethyl) urea
1,6-Di(4-amidinophenoxy)-n-hexane (Hexamidine) and its salts (including isethionate and p-hydroxybenzoate)
Glutaraldehyde (Pentane-1,5-dial)
5-Ethyl-3,7-dioxa-1-azabicyclo[3.3.0]octane
3-(p-chlorophenoxy)-propane-1,2 diol (chlorphenesin)
Silver chloride deposited on titanium dioxide
Benzethonium Chloride (INCI)
Benzalkonium chloride, bromide and saccharinate
Benzylhemiformal
3-Iodo-2-propynylbutylcarbamate
Methylisothiazolinone (INCI)

The chemical structures of compounds a), benzaldehyde and certain benzaldehyde-derivatives, are shown below.

| | |
|---|---|
| Benzaldehyde | 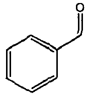 |
| 4-methylbenzaldehyde | 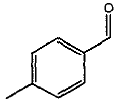 |
| Heliotropine | 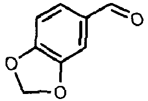 |
| Vanillin | 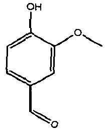 |
| 4-hydroxybenzaldehyde | 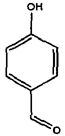 |
| 3-hydroxybenzaldehyde | 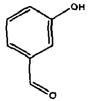 |
| 4-methoxybenzaldehyde | 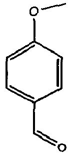 |
| 3-methoxybenzaldehyde | 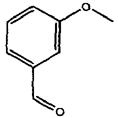 |
| 3-hyd roxy-4-methoxybenzaldehyde (isovanillin) | 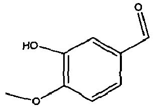 |

These benzaldehyde and benzaldehyde derivative compounds for use as described herein are commercially available.

Compounds b) are fragrant acids selected from cinnamic acid and anisic acid. Their chemical structures are shown below.

| | |
|---|---|
| cinnamic acid | 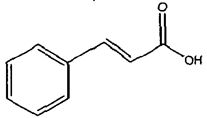 |
| anisic acid | 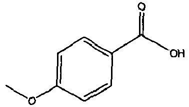 |

### Optional ingredients

Optional ingredients, including optional ingredients to further improve the stabilization against microbial spoilage may be present in personal care products as herein described. In particular, the personal care product may contain one or more aromatic alcohol or derivative thereof as optional ingredient, in particular phenylethyl alcohol, phenyl propyl alcohol, benzyl formiate, and phenethyl formiate. These compounds are commonly used in personal care products as fragrance. Their structural formulae are shown below.

| Aromatic alcohol or derivative | structural formula |
|---|---|
| 2-Phenylethyl alcohol | 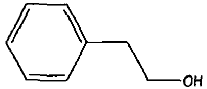 |
| 3-Phenyl propyl alcohol | 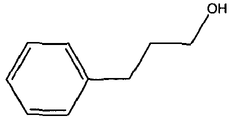 |
| Benzyl formiate | 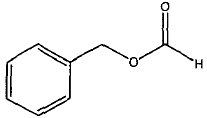 |
| Phenethyl Formiate | 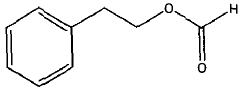 |

Further optional ingredients to further improve the stabilization of the products against microbial spoilage may be antimicrobial (bactericidal and/or fungicidal) fragrance ingredients or compounds.

Examples for the above-mentioned fragrance compounds are given below.

### Antimicrobial fragrant natural oils include but are not limited to:

Geranium oil, Peppermint oil, Rose oil, Cinnamon leaf oil, Fucus oil, Clove bud oil, Clove leaf oil, Palmarosa oil, Citrus Oil, Terpene-fraction from citrus oil, Orange oil, Terpene-fraction from orange oil

### Antimicrobial fragrance compounds found in natural essential oils include but are not limited to:

Geraniol, Cuminic alcohol, Perilla alcohol, Citronellol, Eugenol, Cinnamic alcohol, Nerol, Menthol, Borneol, Octan-1-ol, Nonan-1-ol, Decan-1-ol, Dec-9-en-1-ol, and Limonene.

### Synthetic antimicrobial fragrance alcohols include but are not limited to:

2,6-dimethyl-7-octen-2-ol (Dihydromyrcenol ™),
3-methyl-5-phenyl-pentanol (Phenoxanol ™),
2-methyl-5-phenyl-pentanol (Rosaphen ™),
undec-10-en-1-ol (ALC C11 undecylenique ™),
4-(1-methylethyl)-cyclohexanol (Folrosia ™),
4-(1,1-dimethylethyl)-cyclohexanol (P T Butyl Cyclohexanol ™)
2,2-dimethyl-3-(3-methyl phenyl)-propanol (Majantol™),
4-(1-methylethyl)cyclohexyl-methanol (Mayol™),
(E)-2-(3,3-dimethylbicyclo[2.2.1]hept-2-ylidene)-ethanol (Patchomint™),
3,7-dimethyl-7-octen-1-ol (Rhodinol™),
2-methoxy-4-propyl-phenol (Dihydroeugenol™),
3-(4-methyl-3-cyclohexenyl)-butanol (Cyclomethylencitronellol™),
Dihydroterpineol, and
2,6-dimethylheptan-2-ol (Dimetol™).

### Synthetic antimicrobial fragrance aldehydes include but are not limited to:

3-(4-methoxyphenyl)-2-methyl-Propanal, (Fennaldehyde™)
Octahydro-5-methoxy- 4,7-Methano-1H-indene-2-carboxaldehyde, (Scentenal™)
alpha-methyl-1,3-Benzodioxole-5-propanal, (Tropional™)
3-[4-(1,1-dimethylethyl)phenyl]propanal (Bourgeonal ™)

### Synthetic antimicrobial fragrance lactones include but are not limited to:

8-(1-methylethyl)-1-oxaspiro[4.5]-decan-2-one (Laitone™),
Tetrahydro-6-(2Z)-2-pentenyl-2H-Pyran-2-one, (Lactone Jasmin Delta™)
5-(3Z)-3-hexenyldihydro-2(3H)-Furanone, (Lactone Jasmin Gamma ™)
5-hexyldihydro-5-methyl- 2(3H)-Furanone, (Methyl Decalactone Gamma™), and
4-methyl-5-pentyl-dihydo-2(3H)-Furan-2-one, (Tuberate Methyl Pur™)

### Antimicrobial fragrance lactones found in nature include but are not limited to:

6-hexyltetrahydro-2H-pyran-2-one (Decalactone Delta™),
5-hexyl-Furan-2(3H)-one (Decalactone Gamma ™)
6-heptyltetrahydro-2H-Pyran-2-one, (Dodecalactone Delta™)
5-octyl -furan-2-one, (Dodecalactone Gamma ™)
Dihydro-5-pentyl-2(3H)-Furanone, (Prunolide ™)
5-heptyldihydro-(2(3H)-Furanone), (Gamma-Undecalactone™)
6-hexyltetrahydro- 2H-Pyran-2-one, (Delta-Undecalactone™)

The antimicrobial fragrance compounds may be synthesized or extracted from natural sources including but not limited to botanical sources, for examples fragrant natural oils derived from plants or part of plants, including but not limited to Geranium oil, Peppermint oil, Rose oil, Cinnamon leaf oil, Fucus oil, Clove bud oil, Clove leaf oil, Palmarosa oil, Citrus oil (for example: terpene fraction), and Orange oil (for example: terpene fraction).

Bases for personal care products are well known in the art and the resulting personal care product will usually have a pH of pH5 to pH9 (for example, slightly acidic for products applied to and left on the skin, slightly alkaline for soap products). It is also possible to employ an existing preservative-free personal care product composition and simply add a) and b) in the concentrations hereinabove defined and mix thoroughly.

The exact concentration of compounds under a) and b) that is employed in a composition will depend upon the nature of the product, the stabilisation effect against microbial spoilage (in particular the bactericidal, fungicidal and sporicidal activity), and the length of this effect to be achieved.

A useful concentration for the compound a) is, for example, without limitation, 0.05 to 0.5%, 0.075 to 0.3%, and 0.1 to 0.2% (w/w).
A useful concentration for the compound b) is, for example, 0.05 to 0.5%, 0.075 to 0.3%, and 0.1 to 0.2% (w/w).
If combined in the given concentrations, compounds a) and b) generally provide a sufficient bactericidal, fungicidal and sporicidal activity in a wide range of personal care product compositions.

In particular, a sufficient bactericidal activity is attained when the reduction factor is 1000 per 7 days.
A sufficient sporicidal activity is attained when the reduction factor is 100 per 7 days. A sufficient sporicidal activity is strongly indicative of a sufficient fungicidal activity.
The reduction factor is determined by growing a suitable test organism (*Aspergillus niger* for fungi, *Pseudomonas aeruginosa* for gram-negative bacteria and *Staphylococcus aureus* for gram-positive bacteria) on a suitable culture medium on agar plates, harvesting and adding to a personal care product composition in a density of 3x10⁵ organism/ml and counting the surviving organisms in the probe and a negative control at defined time interval, commonly 7 days. The count of the negative control is divided by the count of the probe and thereby the reduction factor is determined (compare example 1).

b)-compounds of particular interest are 4-hydroxybenzaldehyde and 3-hydroxybenzaldehyde, for their surprisingly good activity.
The addition of hydroxy groups to benzaldehyde and derivatives was previously shown not to provide a fungicidal effect on A. niger.

Fitzgerald et al (who looked at fungistatic effects only, and only of certain food-relevant fungi excluding A. niger), found that the removal of hydroxy groups from 4-hydroxybenzaldehyde resulted in a slight improvement of fungistatic activity against certain food molds, and the only position benefitial for antifungal (fungistatic) activity was the 2-OH position within the benzene ring of benzaldehyde (J. Agric. Food Chem. 2005, 53,1769-1775).

Personal care product compositions are used to form a personal care product in an appropriate application form and packaging, as is well-known in the art.

Personal care products and compositions to form them as decribed herein are used for the purpose of cleansing, conditioning, grooming, beautifying, promoting attractiveness, or otherwise enhancing or altering the appearance of the human body and are applied to the human skin or scalp.
These include products applied to and left on the skin or scalp, for example creams, salves, lotions, and ointments for hand, face or body, perfumes, eau de Cologne, eau de toilet, deodorants, antiperspirants, and products applied but rinsed off such as soaps, liquid soaps, shower gels, shampoos.
These products can, for example, take various forms of application, for example sticks, roll-ons, sprays, pump-sprays, aerosols, soap bars, powders, solutions, gels, creams, balms and lotions.

Many personal care products will be formulated as an emulsion or other lipid-containing products and these form a particular aspect of the embodiments described-herein. Lipids are often included for example into washing formulations including liquid soaps or washing lotions to provide an oil replenishing effect. The a) and b) compounds as hereinabove defined allow the formulation of stabilized emulsions or formulations comprising lipids and/or detergents where the activity (the bactericidal, fungicidal and in particular the sporicidal effect) is not lost due to the presence of the lipid base and/or detergents or surfactants.

Depending on the nature of the personal care product, personal care product compositions as described herein may also be combined with art-recognised quantities of other excipients commonly employed in these products; useful selections may be found in « CTFA Cosmetic Ingredient Handbook », J.M. Nikitakis (ed.), 1st ed., The Cosmetic, Toiletry and Fragrance Association, Inc., Washington, 1988, which is hereby incorporated by reference.

In general, excipients may, for example, include colorants, fragrances, solvents, surfactants, colorants, opacifiers, buffers, antioxidants, vitamins, emulsifiers, UV absorbers, silicones and the like. All products can also be buffered to the desired pH using commonly-available excipients in a known manner.
There now follows a series of non-limiting examples that serve to illustrate the invention.

While the personal care product compositions, products, and related methods have been described above in connection with certain illustrative embodiments, it is to be understood that other similar embodiments may be used or modifications and additions may be made to the described embodiments for performing the same function. Further, all embodiments disclosed are not necessarily in the alternative, as various embodiments may be combined to provide the desired characteristics. Variations can be made by one having ordinary skill in the art without departing from the scope of the disclosure. Therefore, the compositions, products and methods should not be limited to any single embodiment, but rather construed in breadth and scope in accordance with the recitation of the attached claims.

### Examples

Unless otherwise indicated, all concentrations given are % w/w.

### Example 1

### Sporicidal effect of test compounds in water

*Aspergillus niger* ATCC 16404 spores are added to water to obtain a density of 3 × 10⁵ spores / ml.
In order to prepare the spores, the test strain is grown for 5 days on potato dextrose agar at room temperature. The spores are harvested with a solution containing 0.1% Tween 80, peptone 0.1% and NaCl 0.85% and the spore concentration is adjusted to the density indicated above.
Test compounds are dissolved in dipropyleneglycol to a concentration of 20%.
These stock solutions are added to 10 ml aliquots of the spore suspension to obtain a final concentration of the test compounds of 0.1%. The sporicidal effect is shown by a reduction of spore counts after 7 days.

The reduction factor is determined as follows. Aliquots of the above prepared suspension of microorganisms (here: spore suspension prepared as described above) are plated on a suitable agar medium (see above) and the developing colonies are counted both for samples with test compound and for a negative probe (water). The count of the negative control is divided by the count of the test compound and thereby the reduction factor is determined. A negative control (water) accordingly has a reduction factor of 1 (no effect on the microorganism).

**Table 1 Sporicidal effect of test compounds in water**

| **Test compound** | **0.1% test compound** | |
|---|---|---|
| | **Spores / ml** | **Reduction factor** |
| Negative control (water) | 2.5 × 10⁵ | 1 |
| 4-hydroxybenzaldehyde | > 1.2 × 10⁵ | < 2 |
| 4-methoxy-benzaldehyde | 4.68 × 10⁴ | 5 |

No significant reduction of spore counts is achieved with 4-hydroxybenzaldehyde and 4-methoxy-benzaldehyde.

### Example 2

### Sporicidal effect of a) and b) compounds in a cosmetic cream

A preservative-free cosmetic cream for application to the human skin is used (Cremor basalis, Fagron GmbH, Barsbüttel, Germany).

Test samples of cream contain different amounts of compounds a) and b) added to an aliquot of 10 g of the cream in 50 ml tubes to give a concentration (w/w) of 0.125-0.25% as shown in the table below. After addition of the compounds a) and b), the cream is thoroughly mixed to achieve a homogeneous distribution.

After 1 - 3 days of equilibration of the cream (storage at room temperature to achieve a homogenous partitioning of compounds between oil and water phase), to each sample 100 µl of a spore suspension of *Aspergillus niger* ATCC 16404 containing 3 × 10⁷ spores / ml (prepared as described in example 1) is added. After regular test intervals (7 days), samples of 1 g cream are removed and added to 20 ml of a neutralizer solution containing 0.2% lecithin, 2 % Tween 80 and 0.5% NaCl. These dilutions are vigorously shaken for 10 min until the cream is dissolved, and then aliquots of this solution are spread plated on potato dextrose agar containing 0.2% Tween 80. After a sufficient time for colonies to grow (about 48 h), the number of colony forming units (and therefore surviving spores in the cream) are counted.

The table below shows the reduction factor after 7 days of A. niger spores of anisic acid or cinnamic acid (in form of their sodium salts) when combined with 4-hydroxybenzaldehyde (4-HBA) in a skin cream.

**Table 1 Sporicidal effect of cosmetic cream samples with anisic acid and cinnamic acid alone or in combination with 4-HBA.**

| **Fragrant Acid** | **Concentration Fragrant Acid [% w/w]** | **Concentration 4-HBA [% w/w]** | **Reduction factor (*)** |
|---|---|---|---|
| none | 0 | 0.125 | 5.5 |
| Anisic acid | 0.250 | 0 | 12.5 |
| Anisic acid | 0.250 | 0.125 | > 1000 |
| Anisic acid | 0.125 | 0 | 1.4 |
| Anisic acid | 0.125 | 0.125 | > 1000 |
| Cinnamic acid | 0.250 | 0 | 1.1 |
| Cinnamic acid | 0.250 | 0.125 | > 1000 |
| Cinnamic acid | 0.125 | 0 | 1.0 |
| Cinnamic acid | 0.125 | 0.125 | 225.8 |

Surprisingly, though 4-hydroxybenzaldehyde shows no significant sporicidal effect in water (compare example1), this and several other test compounds show an excellent activity when combined with cinnamic acid or anisic acid in cream at 0.125% concentration (complete killing of spores within 7days).

The results show that the sporicidal effect on *Aspergillus niger* spores is only achieved when combining anisic acid or cinnamic acid with 4-HBA. Neither one of the fragrant acids alone achieves the sporicidal effect necessary for stabilisation of personal care products (reduction factors of maximally up to 12.5). Nor does 4-HBA achieve a significant sporicidal effect in cosmetic cream, at 0.125% concentration the reduction factor is 5.5.

Similar results are achieved when employing, instead of 4-HBA, another a) compound selected from Benzaldehyde, 4-methylbenzaldehyde, Heliotropine, Vanillin, 4-hydroxybenzaldehyde, 3-hydroxybenzaldehyde, 4-methoxybenzaldehyde, 3-methoxybenzaldehyde, and 3-hydroxy-4-methoxybenzaldehyde (isovanillin).

### Example 3

### Bactericidal effect of a) and b) compounds in a cosmetic cream

Test samples of the same cream used in Example 2 contain different amounts of compounds a) and b) added to an aliquot of 10 g of the cream in 50 ml tubes to give a concentration (w/w) of 0.125-0.25% as shown in the table below. After addition of the compounds a) and b), the cream is thoroughly mixed to achieve a homogeneous distribution.

As test organisms *Staphylococcus aureus* (DSMZ 799) and *Pseudomonas aeruginosa* (ATCC 15442) are used. The strains are grown overnight in Mueller-Hinton broth and adjusted to a cell density of 1 x 10⁸ cfu (colony forming units) per ml.
The two bacterial strains are mixed in a ratio of 1:1 and 100 µl of this mixed inoculum is added to 10 ml aliquots of the cosmetic cream.

The resulting mixtures are incubated at room temperature and at the regular intervals samples are removed, suspended in neutralizer solution and diluted as described above (example 2).

Aliquots of these suspended and diluted samples are plated on tryptic soy agar supplemented with 0.5% Tween 80 and then incubated for 24 h at 37°C. Surviving bacteria are counted.

The table below shows the reduction factor of bacterial counts after 24 h for anisic acid or cinnamic acid (in form of their sodium salts) when combined with 4-hydroxybenzaldehyde (4-HBA) in a skin cream.

**Table 2 bactericidal effect of cosmetic cream samples with anisic acid and cinnamic acid alone or in combination with 4-HBA.**

| **Fragrant Acid** | **Concentration Fragrant Acid [% w/w]** | **Concentration 4-HBA [% w/w]** | **Reduction factor (*)** |
|---|---|---|---|
| none | | 0.125 | 10 |
| Anisic acid | 0.250 | 0 | 5 |
| Anisic acid | 0.250 | 0.125 | > 1000 |
| Anisic acid | 0.125 | 0 | 1 |
| Anisic acid | 0.125 | 0.125 | > 1000 |
| Cinnamic acid | 0.250 | 0 | 10 |
| Cinnamic acid | 0.250 | 0.125 | > 1000 |
| Cinnamic acid | 0.125 | 0 | 2.0 |
| Cinnamic acid | 0.125 | 0.125 | > 1000 |

Similar results are achieved when employing, instead of 4-HBA, another a) compound selected from Benzaldehyde, 4-methylbenzaldehyde, Heliotropine, Vanillin, 4-hydroxybenzaldehyde, 3-hydroxybenzaldehyde, 4-methoxybenzaldehyde, 3-methoxybenzaldehyde, and 3-hydroxy-4-methoxybenzaldehyde (isovanillin).

These results show that bactericidal effects to prevent the product from microbial spoilage can be achieved by combining a compound selected from the compound a) with a compound selected from the compounds b).

## Claims

1. A personal care product composition comprising
a) at least one benzaldehyde or benzaldehyde-derivative compound of formula I, 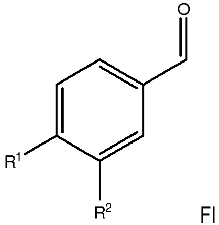
wherein R¹ is a residue selected from the group consisting of H, CH₂, CH₃, OH, and OCH₃,
wherein R² is a residue selected from the group consisting of H, OH, OCH₃, and OCH₂O, and
wherein when R² is OCH₂O, then R¹ is CH₂ and forms a bond with R², selected from the group consisting of 4-methylbenzaldehyde, Heliotropine, Vanillin, 4-hydroxybenzaldehyde, 3-hydroxybenzaldehyde, 3-methoxybenzaldehyde, and 3-hydroxy-4-methoxybenzaldehyde (isovanillin); and
b) at least one fragrant acid according to formula II 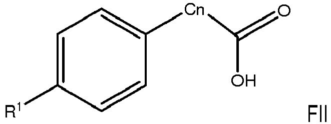
wherein R¹ is a residue selected from the group consisting of H and OCH₃,
wherein n is selected from 0 or 2, and
wherein if R¹ is H then n is 2, and the C atoms of the resulting C₂ situated between aromatic ring and COOH form a double bond, and
wherein if R¹ is OCH₃ then n is 0;
selected from the group consisting of cinnamic acid and anisic acid;
wherein the at least one benzaldehyde or benzaldehyde-derivative compound a) is present in a total concentration of 0.05 to 0.5% (w/w);
wherein the at least one fragrant acid b) is present in a total concentration of 0.05 to 0.5% (w/w);
and a cosmetically-acceptable base,
with the proviso that the personal care product composition is essentially free of a preservative selected from the group consisting of Formaldehyde paraformaldehyde, Biphenyl-2-ol (o-phenylphenol) and its salts, Pyrithione zinc, Inorganic sulphites and hydrogen- sulphites, Sodium iodate, Chlorobutanol, 4-Hydroxybenzoic acid and its salts and esters, 3-Acetyl-6-methylpyran-2,4 (3H)-dione (Dehydracetic acid) and its salts, Formic acid and its sodium salt, 3,3'-Dibromo-4,4'-hexamethylenedioxydibenzamidine (Dibromohexamidine) and its salts (including isethionate), Thiomersal, Phenylmercuric salts (including borate), Hexetidine, 5-Bromo-5-nitro-1,3-dioxane, Bronopol, 2,4-Dichlorobenzyl alcohol, Triclocarban, 4-Chloro-m-cresol, 4-Chloro-3,5-xylenol, 3,3'-Bis (1-hydroxymethyl-2,5-dioxoimidazolidin-4-yl)-1,1'-methylenediurea ('Imidazolidinyl urea'), Hexamethylenetetramine (methenamine), Methenamine 3-chloroallylochloride, 1-(4-Chlorophenoxy)-1-(imidazol-1-yl)-3,3-dimethylbutan-2-one, 1,3-Bis (hydroxymethyl)-5,5-dimethylimidazolidine-2,4-dione, 1-Hydroxy-4-methyl-6(2,4,4-trimethylpentyl) 2-pyridon and its monoethanolamine salt , 1,2-Dibromo-2,4-dicyanobutane (methyldibromoglutaronitrile), 6,6-Dibromo-4,4-dichloro2,2'-methylenediphenol (Bromochlorophen), Mixture of 5-Chloro-2-methyl-isothiazol-3 (2H)-one and 2-methylisothiazol-3(2H)-one with magnesium chloride and magnesium nitrate, 2-Benzyl-4-chlorophenol (chlorophene), 2-Chloroacetamide, Chlorhexidine and its digluconate, diacetate and dihydrochloride, Alkyl (C12-C22) trimethyl ammonium, bromide and chloride, 4,4-dimethyl-1,3-oxizalidine, N-(Hydroxymethyl)-N-(dihydroxymethyl-1,3-dioxo-2,5-imidazolidinyl-4)-N'-(hydroxymethyl) urea, 1,6-Di (4-amidinophenoxy)-n-hexane (Hexamidine) and its salts (including isethionate and p-hydroxybenzoate), Glutaraldehyde (Pentane-1,5-dial), 5-Ethyl-3,7-dioxa-1-azabicyclo [3.3.0]octane, 3-(p-chlorophenoxy)-propane-1,2 diol (chlorphenesin), Silver chloride deposited on titanium dioxide, Benzethonium Chloride, Benzalkonium chloride, bromide and saccharinate, Benzylhemiformal , 3-lodo-2-propynylbutylcarbamate , and Methylisothiazolinone
**characterized in that** the composition comprises:
as component (a) 4-hydroxybenzaldehyde; and
as component (b) anisic acid.

2. The personal care product composition according to claim 1 wherein the at least one benzaldehyde or benzaldehyde-derivative compound a) is present in a concentration of 0.075 to 0.3% (w/w), optionally 0.1 to 0.2%, (w/w), and wherein the at least one fragrant acid b) is present in a concentration of 0.075 to 0.3% (w/w), optionally 0.1 to 0.2% (w/w).

3. The personal care product composition according to any one of claims 1 and 2 further comprising at least one aromatic alcohol or derivative thereof selected from the group consisting of phenylethyl alcohol, phenyl propyl alcohol, benzyl formiate, and phenethyl formiate.

4. The personal care product composition according to any one of claims 1 and 2 further comprising an antimicrobial fragrance ingredient selected from the group consisting of Geranium oil, Peppermint oil, Rose oil, Cinnamon leaf oil, Fucus oil, Clove bud oil, Clove leaf oil, Palmarosa oil, Citrus oil, Terpene fraction of citrus oil, Orange oil, Terpene fraction of orange oil, Geraniol, Cuminic alcohol, Perilla alcohol, Citronellol, Eugenol, Cinnamic alcohol, Nerol, Menthol, Borneol, Octan-1-ol, Nonan-1-ol, Decan-1-ol, Dec-9-en-1-ol, Limonene, 2,6-dimethyl-7-octen-2-ol, 3-methyl-5-phenyl-pentanol, 2-methyl-5-phenyl-pentanol, undec-10-en-1-ol, 4-(1-methylethyl)-cyclohexanol, 4-(1,1-dimethylethyl)-cyclohexanol, 2,2-dimethyl-3-(3-methyl phenyl)-propanol, 4-(1-methylethyl)cyclohexyl-methanol, (E)-2-(3,3-dimethylbicyclo[2.2.1]hept-2-ylidene)-ethanol, 3,7-dimethyl-7-octen-1-ol, 2-methoxy-4-propyl-phenol, 3-(4-methyl-3-cyclohexenyl)-butanol, Dihydroterpineol, 2,6-dimethylheptan-2-ol, 3-(4-methoxyphenyl)-2-methyl- Propanal, Octahydro-5-methoxy- 4,7-Methano-1H-indene-2-carboxaldehyde, alpha-methyl-1,3-Benzodioxole-5-propanal, 3-[4-(1,1-dimethylethyl)phenyl]propanal, 8-(1-methylethyl)-1-oxaspiro[4.5]-decan-2-one, Tetrahydro-6-(2Z)-2-pentenyl-2H-Pyran-2-one, 5-(3Z)-3-hexenyldihydro-2(3H)-Furanone, 5-hexyldihydro-5-methyl- 2(3H)-Furanone, 4-methyl-5-pentyl-dihydo-2(3H)-Furan-2-one, 6-hexyltetrahydro-2H-pyran-2-one, 5-hexyl-Furan-2(3H)-one, 6-heptyltetrahydro-2H-Pyran-2-one, 5-octyl -furan-2-one, Dihydro-5-pentyl-2(3H)-Furanone, 5-heptyldihydro- (2(3H)-Furanone), and 6-hexyltetrahydro- 2H-Pyran-2-one.

5. The personal care product composition according to any one of claims 1 and 2 further comprising at least one aromatic alcohol or derivative thereof as defined in claim 3 and at least one antimicrobial fragrance ingredient as defined in claim 4.

6. A composition according to any one of claims 1 to 5 selected from compositions for personal care products applied to and left on the skin or scalp including but not limited to creams, salves, lotions, and ointments for hand, face or body, perfumes, eau de Cologne, eau de toilet, deodorants, antiperspirants, and products applied but rinsed off including but not limited to soaps, liquid soaps, shower gels, and shampoos.

7. Use of at least one benzaldehyde or benzaldehyde-derivative compound a) as defined in any one of the claims 1 to 6 and at least one fragrant acid b) as defined in any one of the claims 1 to 6 for the preparation of a preserved personal care product composition as defined in any one of claims 1-6.

8. Method of forming a stabilized personal care product composition as defined in any one of claims 1-6 which is sufficiently bactericidal to have a reduction factor for Pseudomonas aeruginosa and Staphylococcus aureus of at least 1000 per 7 days, and which is sufficiently sporicidal to have a reduction factor of at least 100 per 7 days for Aspergillus niger,
by admixing an effective amount of at least one benzaldehyde or benzaldehyde-derivative compound a) and an effective amount of at least one fragrant acid b) as defined in any one of claims 1 to 6 to a personal care product base, wherein optionally said compounds a) and b) may be added in form of a pre-prepared fragrance composition comprising said compounds a) and b), forming a personal care product composition..

9. Method according to claim 8 wherein the total concentration of the at least one benzaldehyde or benzaldehyde-derivative compound a) in the personal care product composition is from 0.3 to 0.5%, and the total concentration of the at least one fragrant acid b) in the personal care product composition is from 0.3 to 0.5%.

10. Method of any one of claims 8 and 9 wherein the personal care product composition formed further comprises at least one aromatic alcohol or derivative thereof selected from the group consisting of phenylethyl alcohol, phenyl propyl alcohol, benzyl formiate, and phenethyl formiate.

11. Method of any one of claims 8 and 9 wherein the personal care product composition formed further comprises at least one antimicrobial fragrance ingredient as defined in claim 4.

12. Method of any one of claims 8 and 9 wherein the personal care product composition formed further comprises at least one aromatic alcohol or derivative thereof selected from the group consisting of phenylethyl alcohol, phenyl propyl alcohol, benzyl formiate, and phenethyl formiate; and at least one antimicrobial fragrance ingredient as defined in claim 4.

13. Method of any one of claims 8 and 9 wherein the personal care product composition is or is brought into the form of a personal care product including sticks, roll-ons, sprays, pump-sprays, aerosols, soap bars, powders, solutions, gels, creams, balms and lotions.

## Patentansprüche

1. Körperpflegeproduktzusammensetzung, umfassend
a) mindestens eine Benzaldehyd- oder Benzaldehydderivat-Verbindung der Formel I, 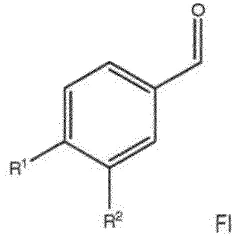
wobei R¹ für einen Rest aus der Gruppe bestehend aus H, CH₂, CH₃, OH und OCH₃ steht,
wobei R² für einen Rest aus der Gruppe bestehend aus H, OH, OCH₃ und OCH₂O steht, und
wobei dann, wenn R² für OCH₂O steht, R¹ für CH₂ steht und eine Bindung mit R² bildet, ausgewählt aus der Gruppe bestehend aus 4-Methylbenzaldehyd, Heliotropin, Vanillin, 4-Hydroxybenzaldehyd, 3-Hydroxybenzaldehyd, 3-Methoxybenzaldehyd und 3-Hydroxy-4-methoxybenzaldehyd (Isovanillin); und
b) mindestens eine duftende Säure gemäß Formel II 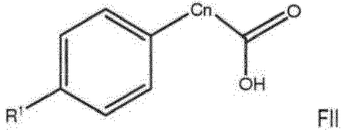
wobei R¹ für einen Rest aus der Gruppe bestehend aus H und OCH₃ steht,
wobei n aus 0 oder 2 ausgewählt ist, und
wobei dann, wenn R¹ für H steht, n für 2 steht und die C-Atome des resultierenden C₂ zwischen dem aromatischen Ring und dem COOH eine Doppelbindung bilden, und
wobei dann, wenn R¹ für OCH₃ steht, n für 0 steht; ausgewählt aus der Gruppe bestehend aus Zimtsäure und Anissäure;
wobei die mindestens eine Benzaldehyd- oder Benzaldehydderivat-Verbindung a) in einer Gesamtkonzentration von 0,05 bis 0,5% (w/w) vorliegt; wobei die mindestens eine duftende Säure b) in einer Gesamtkonzentration von 0,05 bis 0,5% (w/w) vorliegt;
und eine kosmetisch unbedenkliche Basis,
mit der Maßgabe, dass die Körperpflegeproduktzusammensetzung im Wesentlichen frei von einem Konservierungsstoff aus der Gruppe bestehend aus Formaldehyd, Paraformaldehyd, Biphenyl-2-ol (o-Phenylphenol) und Salzen davon, Pyrithion-Zink, anorganischen Sulfiten und Hydrogensulfiten, Natriumiodat, Chlorbutanol, 4-Hydroxybenzoesäure und Salzen und Estern davon, 3-Acetyl-6-methyl-pyran-2,4(3H)-dion (Dehydracetsäure) und Salzen davon, Ameisensäure und deren Natriumsalz, 3,3'-Dibrom-4,4'-hexamethylendioxydibenzamidin (Dibromhexamidin) und Salzen davon (einschließlich Isethionat), Thiomersal, Phenylquecksilbersalzen (einschließlich Borat), Hexetidin, 5-Brom-5-nitro-1,3-dioxan, Bronopol, 2,4-Dichlorbenzylalkohol, Triclocarban, 4-Chlor-m-kresol, 4-Chlor-3,5-xylenol, 3,3'-Bis(1-hydroxymethyl-2,5-dioxo-imidazolidin-4-yl)-1,1'-methylendiharnstoff ("Imidazolidinylharnstoff"), Hexamethylentetraamin (Methenamin), Methenamin-3-chlorallylochlorid, 1-(4-Chlorphenoxy)-1-(imidazol-1-yl)-3,3-dimethylbutan-2-on, 1,3-Bis(hydroxymethyl)-5,5-dimethyl-imidazolidin-2,4-dion, 1-Hydroxy-4-methyl-6-(2,4,4-trimethylpentyl)-2-pyridon und dessen Monoethanolaminsalz, 1,2-Dibrom-2,4-dicyanobutan (Methyldibromglutaronitril), 6,6-Dibrom-4,4-dichlor-2,2'-methylendiphenol (Bromchlorophen), Mischung von 5-Chlor-2-methylisothiazol-3(2H)-on und 2-Methylisothiazol-3(2H)-on mit Magnesiumchlorid und Magnesiumnitrat, 2-Benzyl-4-chlorphenol (Chlorophen), 2-Chloracetamid, Chlorhexidin und dessen Digluconat, Diacetat und Dihydrochlorid, Alkyl(C12-C22)trimethylammoniumbromid und -chlorid, 4,4-Dimethyl-1,3-oxizalidin, N-(Hydroxymethyl)-N-(dihydroxymethyl-1,3-dioxo-2,5-imidazolidinyl-4)-N'-(hydroxymethyl)harnstoff, 1,6-Di(4-amidinophenoxy)n-hexan (Hexamidin) und Salzen davon (einschließlich Isethionat und p-Hydroxybenzoat), Glutaraldehyd (Pentan-1,5-dial), 5-Ethyl-3,7-dioxa-1-azabicyclo[3.3.0]octan, 3-(p-Chlorphenoxy)propan-1,2-diol (Chlorphenesin), auf Titandioxid abgeschiedenem Silberchlorid, Benzethoniumchlorid, Benzalkoniumchlorid, -bromid und -saccharinat, Benzylhemiformal, 3-Iod-2-propinylbutylcarbamat und Methylisothiazolinon ist,
**dadurch gekennzeichnet, dass** die Zusammensetzung als Komponente (a) 4-Hydroxybenzaldehyd und als Komponente (b) Anissäure umfasst.

2. Körperpflegeproduktzusammensetzung nach Anspruch 1, wobei die mindestens eine Benzaldehyd- oder Benzaldehydderivat-Verbindung a) in einer Konzentration von 0,075 bis 0,3 % (w/w), gegebenenfalls von 0,1 bis 0,2 % (w/w) vorliegt und wobei die mindestens eine duftende Säure b) in einer Konzentration von 0,075 bis 0,3 % (w/w), gegebenenfalls von 0,1 bis 0,2 % (w/w) vorliegt.

3. Körperpflegeproduktzusammensetzung nach einem der Ansprüche 1 und 2, ferner umfassend mindestens einen aromatischen Alkohol oder ein Derivat davon aus der Gruppe bestehend aus Phenylethylalkohol, Phenylpropylalkohol, Ameisensäurebenzylester und Ameisensäurephenethylester.

4. Körperpflegeproduktzusammensetzung nach einem der Ansprüche 1 und 2, ferner umfassend einen antimikrobiellen Duftstoffbestandteil aus der Gruppe bestehend aus Geraniumöl, Pfefferminzöl, Rosenöl, Zimtblätteröl, Fucusöl, Nelkenknospenöl, Nelkenblätteröl, Palmarosaöl, Citrusöl, Terpenfraktion von Citrusöl, Orangenöl, Terpenfraktion von Orangenöl, Geraniol, Cuminalkohol, Perillaalkohol, Citronellol, Eugenol, Zimtalkohol, Nerol, Menthol, Borneol, Octan-1-ol, Nonan-1-ol, Decan-1-ol, Dec-9-en-1-ol, Limonen, 2,6-Dimethyl-7-octen-2-ol, 3-Methyl-5-phenylpentanol, 2-Methyl-5-phenyl-pentanol, Undec-10-en-1-ol, 4-(1-Methylethyl)-cyclohexanol, 4-(1,1-Dimethylethyl)cyclohexanol, 2,2-Dimethyl-3-(3-methylphenyl)propanol, 4-(1-Methyl-ethyl)cyclohexylmethanol, (E)-2-(3,3-Dimethylbicyclo[2.2.1]hept-2-yliden)ethanol, 3,7-Dimethyl-7-octen-1-ol, 2-Methoxy-4-propylphenol, 3-(4-Methyl-3-cyclohexenyl)butanol, Dihydroterpineol, 2,6-Dimethylheptan-2-ol, 3-(4-Methoxyphenyl)-2-methylpropanal, Octahydro-5-methoxy-4,7-methano-1H-inden-2-carboxaldehyd, alpha-Methyl-1,3-benzodioxol-5-propanal, 3-[4-(1,1-Dimethylethyl)phenyl]propanal, 8-(1-Methylethyl)-1-oxa-spiro[4.5]decan-2-on, Tetrahydro-6-(2Z)-2-pentenyl-2H-pyran-2-on, 5-(3Z)-3-Hexenyldihydro-2(3H)-furanon, 5-Hexyldihydro-5-methyl-2(3H)-furanon, 4-Methyl-5-pentyldihydro-2(3H)-furan-2-on, 6-Hexyltetrahydro-2H-pyran-2-on, 5-Hexylfuran-2(3H)-on, 6-Heptyltetrahydro-2H-pyran-2-on, 5-Octylfuran-2-on, Dihydro-5-pentyl-2(3H)-furanon, 5-Heptyldihydro(2(3H)-furanon) und 6-Hexyltetrahydro-2H-pyran-2-on.

5. Körperpflegeproduktzusammensetzung nach einem der Ansprüche 1 und 2, ferner umfassend mindestens einen aromatischen Alkohol oder ein Derivat davon gemäß Anspruch 3 und mindestens einen antimikrobiellen Duftstoffbestandteil gemäß Anspruch 4.

6. Zusammensetzung nach einem der Ansprüche 1 bis 5, ausgewählt aus Zusammensetzungen für Körperpflegeprodukte, die auf die Haut oder Kopfhaut aufgebracht und darauf belassen werden, einschließlich u. a. Cremes, Unguenta, Lotionen und Salben für Hände, Gesicht oder Körper, Parfümen, Eau de Cologne, Eau de Toilette, Deodorantien, Antitranspirantien, und Produkte, die auf die Haut oder Kopfhaut aufgetragen, aber abgespült werden, einschließlich u. a. Seifen, Flüssigseifen, Duschgelen und Shampoos.

7. Verwendung mindestens einer Benzaldehyd- oder Benzaldehydderivat-Verbindung a) gemäß einem der Ansprüche 1 bis 6 und mindestens einer duftenden Säure b) gemäß einem der Ansprüche 1 bis 6 zur Herstellung einer konservierten Körperpflegeproduktzusammensetzung gemäß einem der Ansprüche 1-6.

8. Verfahren zur Bildung einer stabilisierten Körperpflegeproduktzusammensetzung gemäß einem der Ansprüche 1-6, die so bakterizid ist, dass sie einen Reduktionsfaktor für Pseudomonas aeruginosa und Staphylococcus aureus von mindestens 1000 pro 7 Tage aufweist, und so sporizid ist, dass sie einen Reduktionsfaktor von mindestens 100 pro 7 Tage für Aspergillus niger aufweist,
durch Beimischen einer wirksamen Menge mindestens einer Benzaldehyd- oder Benzaldehydderivat-Verbindung a) und einer wirksamen Menge mindestens einer duftenden Säure b) gemäß einem der Ansprüche 1 bis 6 zu einer Körperpflegeproduktgrundlage, wobei gegebenenfalls die Verbindungen a) und b) in Form einer vorher hergestellten Duftstoffzusammensetzung, die die Verbindungen a) und b) umfasst, zugegeben werden können, was eine Körperpflegeproduktzusammensetzung ergibt.

9. Verfahren nach Anspruch 8, wobei die Gesamtkonzentration der mindestens einen Benzaldehyd- oder Benzaldehydderivat-Verbindung a) in der Körperpflegeproduktzusammensetzung 0,3 bis 0,5 % beträgt und die Gesamtkonzentration der mindestens einen duftenden Säure b) in der Körperpflegeproduktzusammensetzung 0,3 bis 0,5 % beträgt.

10. Verfahren nach einem der Ansprüche 8 und 9, wobei die gebildete Körperpflegeproduktzusammensetzung ferner mindestens einen aromatischen Alkohol oder ein Derivat davon aus der Gruppe bestehend aus Phenylethylalkohol, Phenylpropylalkohol, Ameisensäurebenzylester und Ameisensäurephenethylester umfasst.

11. Verfahren nach einem der Ansprüche 8 und 9, wobei die gebildete Körperpflegeproduktzusammensetzung ferner mindestens einen antimikrobiellen Duftstoffbestandteil gemäß Anspruch 4 umfasst.

12. Verfahren nach einem der Ansprüche 8 und 9, wobei die gebildete Körperpflegeproduktzusammensetzung ferner mindestens einen aromatischen Alkohol oder ein Derivat davon aus der Gruppe bestehend aus Phenylethylalkohol, Phenylpropylalkohol, Ameisensäurebenzylester und Ameisensäurephenethylester und mindestens einen antimikrobiellen Duftstoffbestandteil gemäß Anspruch 4 umfasst.

13. Verfahren nach einem der Ansprüche 8 und 9, wobei die Körperpflegeproduktzusammensetzung in der Form eines Körperpflegeprodukts einschließlich Stiften, Rollern, Sprays, Pumpsprays, Aerosolen, Seifenstücken, Pulvern, Lösungen, Gelen, Cremes, Balsamen und Lotionen vorliegt oder in eine derartige Form gebracht wird.

## Revendications

1. Composition de produit de soin personnel, comprenant
a) au moins un composé de benzaldéhyde ou de dérivé de benzaldéhyde de formule I, 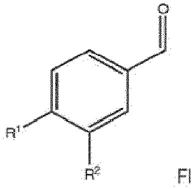
où R¹ est un reste choisi dans le groupe constitué par H, CH₂, CH₃, OH, et OCH₃,
où R² est un reste choisi dans le groupe constitué par H, OH, OCH₃, et OCH₂O, et
où lorsque R² est OCH₂O, alors R¹ est CH₂ et forme une liaison avec R², choisi dans le groupe constitué par le 4-méthylbenzaldéhyde, l'héliotropine, la vanilline, le 4-hydroxybenzaldéhyde, le 3-hydroxybenzaldéhyde, le 3-méthoxybenzaldéhyde, et le 3-hydroxy-4-méthoxy-benzaldéhyde (isovanilline) ; et
b) au moins un acide fragrant répondant à la formule II 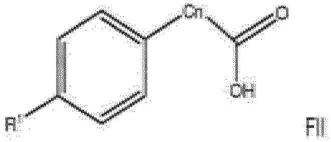
où R¹ est un reste choisi dans le groupe constitué par H et OCH₃,
où n est choisi parmi 0 ou 2, et
où si R¹ est H alors n vaut 2, et les atomes de C du C₂ résultant situés entre le cycle aromatique et COOH forment une double liaison, et
où si R¹ est OCH₃ alors n vaut 0 ;
choisi dans le groupe constitué par l'acide cinnamique et l'acide anisique ;
où le au moins un composé de benzaldéhyde ou de dérivé de benzaldéhyde a) est présent selon une concentration totale allant de 0,05 à 0,5% (p/p) ;
où le au moins un acide fragrant b) est présent selon une concentration totale allant de 0,05 à 0,5% (p/p) ; et une base cosmétiquement acceptable,
à condition que la composition de produit de soin personnel soit essentiellement dépourvue d'un conservateur choisi dans le groupe constitué par le formaldéhyde, le paraformaldéhyde, le biphényl-2-ol (o-phénylphénol) et ses sels, la pyrithione de zinc, les sulfites inorganiques et les hydrogénosulfites, l'iodate de sodium, le chlorobutanol, l'acide 4-hydroxybenzoïque et ses sels et esters, la 3-acétyl-6-méthylpyran-2,4(3H)-dione (acide déshydroacétique) et ses sels, l'acide formique et son sel de sodium, la 3,3'-dibromo-4,4'-hexaméthylènedioxydibenzamidine (dibromohexamidine) et ses sels (y compris l'iséthionate), le thiomersal, les sels phénylmercuriques (y compris le borate), l'hexétidine, le 5-bromo-5-nitro-1,3-dioxane, le bronopol, l'alcool 2,4-dichlorobenzylique, le triclocarban, le 4-chloro-m-crésol, le 4-chloro-3,5-xylénol, la 3,3'-bis(1-hydroxyméthyl-2,5-dioxoimidazolidin-4-yl)-1,1'-méthylènediurée (« imidazolidinylurée »), l'hexa-méthylènetétramine (méthénamine), le 3-chloroallylo-chlorure de méthénamine, la 1-(4-chlorophénoxy)-1-(imidazol-1-yl)-3,3-diméthylbutan-2-one, la 1,3-bis(hydroxyméthyl)-5,5-diméthylimidazolidine-2,4-dione , la 1-hydroxy-4-méthyl-6(2,4,4-triméthylpentyl)-2-pyridone et son sel de monoéthanolamine, le 1,2-dibromo-2,4-dicyanobutane (méthyldibromoglutaro-nitrile), le 6,6-dibromo-4,4-dichloro-2,2'-méthylène-diphénol (bromochlorophène), un mélange de 5-chloro-2-méthyl-isothiazol-3(2H)-one et de 2-méthylisothiazol-3(2H)-one avec du chlorure de magnésium et du nitrate de magnésium, le 2-benzyl-4-chlorophénol (chlorophène), le 2-chloroacétamide, la chlorhexidine et son digluconate, diacétate, et dichlorhydrate, le bromure et le chlorure de (C12-C22)alkyltriméthylammonium, la 4,4-diméthyl-1,3-oxizalidine, la N-(hydroxyméthyl)-N-(dihydroxyméthyl-1,3-dioxo-2,5-imidazolidinyl-4)-N'-(hydroxyméthyl)urée, le 1,6-di(4-amidinophénoxy)-n-hexane (hexamidine) et ses sels (y compris l'iséthionate et le p-hydroxybenzoate), le glutaraldéhyde (pentane-1,5-dial), le 5-éthyl-3,7-dioxa-1-azabicyclo[3.3.0]octane, le 3-(p-chlorophénoxy)-propane-1,2-diol (chlorphénésine), le chlorure d'argent déposé sur du dioxyde de titane, le chlorure de benzéthonium, le chlorure, le bromure et le saccharinate de benzalkonium, le benzylhémiformal, le carbamate de 3-iodo-2-propynylbutyle, et la méthylisothiazolinone,
**caractérisée en ce que** la composition comprend :
comme composant (a), du 4-hydroxybenzaldéhyde ; et
comme composant (b), de l'acide anisique.

2. Composition de produit de soin personnel selon la revendication 1, dans laquelle le au moins un composé de benzaldéhyde ou de dérivé de benzaldéhyde a) est présent selon une concentration allant de 0,075 à 0,3% (p/p), éventuellement de 0,1 à 0,2% (p/p), et où le au moins un acide fragrant b) est présent selon une concentration allant de 0,075 à 0,3% (p/p), éventuellement de 0,1 à 0,2% (p/p).

3. Composition de produit de soin personnel selon l'une quelconque des revendications 1 et 2, comprenant en outre au moins un alcool aromatique ou un dérivé de celui-ci choisi dans le groupe constitué par l'alcool phényléthylique, l'alcool phénylpropylique, le formiate de benzyle, et le formiate de phénéthyle.

4. Composition de produit de soin personnel selon l'une quelconque des revendications 1 et 2, comprenant en outre un ingrédient de fragrance antimicrobien choisi dans le groupe constitué par l'essence de géranium, l'essence de menthe poivrée, l'essence de rose, l'essence de feuilles de cannelle, l'essence de Fucus, l'essence de clous de girofle, l'essence de feuilles de girofle, l'essence de Palmarosa, l'essence de Citrus, la fraction terpénique de l'essence de Citrus, l'essence d'orange, la fraction terpénique de l'essence d'orange, le géraniol, l'alcool cuminique, l'alcool de Perilla, le citronellol, l'eugénol, l'alcool cinnamique, le nérol, le menthol, le bornéol, l'octan-1-ol, le nonan-1-ol, le décan-1-ol, le déc-9-én-1-ol, le limonène, le 2,6-diméthyl-7-octén-2-ol, le 3-méthyl-5-phénylpentanol, le 2-méthyl-5-phényl-pentanol, l'undéc-10-én-1-ol, le 4-(1-méthyléthyl)-cyclohexanol, le 4-(1,1-diméthyléthyl)cyclohexanol, le 2,2-diméthyl-3-(3-méthylphényl)propanol, le 4-(1-méthyléthyl)cyclohexylméthanol, le (E)-2-(3,3-diméthyl-bicyclo[2.2.1]hept-2-ylidène)-éthanol, le 3,7-diméthyl-7-octén-1-ol, le 2-méthoxy-4-propylphénol, le 3-(4-méthyl-3-cyclohexényl)butanol, le dihydroterpinéol, le 2,6-diméthylheptan-2-ol, le 3-(4-méthoxyphényl)-2-méthylpropanal, l'octahydro-5-méthoxy-4,7-méthano-1H-indène-2-carboxaldéhyde, l'alpha-méthyl-1,3-benzo-dioxole-5-propanal, le 3-[4-(1,1-diméthyléthyl)phényl]-propanal, la 8-(1-méthyléthyl)-1-oxaspiro[4.5]-décan-2-one, la tétrahydro-6-(2Z)-2-pentényl-2H-pyran-2-one, la 5-(3Z)-3-hexényldihydro-2(3H)-furanone, la 5-hexyl-dihydro-5-méthyl-2(3H)-furanone, la 4-méthyl-5-pentyl-dihydro-2(3H)-furan-2-one, la 6-hexyltétrahydro-2H-pyran-2-one, la 5-hexyl-furan-2(3H)-one, la 6-heptyl-tétrahydro-2H-pyran-2-one, la 5-octyl-furan-2-one, la dihydro-5-pentyl-2(3H)-furanone, la 5-heptyldihydro-(2(3H)-furanone), et la 6-hexyltétrahydro-2H-pyran-2-one.

5. Composition de produit de soin personnel selon l'une quelconque des revendications 1 et 2, comprenant en outre au moins un alcool aromatique ou un dérivé de celui-ci tel que défini selon la revendication 3 et au moins un ingrédient de fragrance antimicrobien tel que défini selon la revendication 4.

6. Composition selon l'une quelconque des revendications 1 à 5, choisie parmi les compositions pour des produits de soin personnel appliqués sur la peau ou le cuir chevelu et laissés sur ceux-ci, y compris, sans y être limités, les crèmes, les pommades, les lotions et les onguents pour les mains, le visage ou le corps, les parfums, l'eau de Cologne, l'eau de toilette, les déodorants, ou antitranspirants ; et les produits appliqués mais éliminés par rinçage, y compris, sans y être limités, les savons, les savons liquides, les gels douche et les shampooings.

7. Utilisation d'au moins un composé de benzaldéhyde ou de dérivé de benzaldéhyde a) tel que défini selon l'une quelconque des revendications 1 à 6 et d'au moins un acide fragrant b) tel que défini selon l'une quelconque des revendications 1 à 6, pour la préparation d'une composition de produit de soin personnel préservée telle que définie selon l'une quelconque des revendications 1-6.

8. Méthode de formation d'une composition de produit de soin personnel stabilisée telle que définie selon l'une quelconque des revendications 1-6, qui est suffisamment bactéricide pour posséder un facteur de réduction pour *Pseudomonas aeruginosa* et *Staphylococcus aureus* d'au moins 1000 pour 7 jours, et qui est suffisamment sporicide pour posséder un facteur de réduction d'au moins 100 pour 7 jours pour *Aspergillus niger,*
par le mélange d'une quantité efficace d'au moins un composé de benzaldéhyde ou de dérivé de benzaldéhyde a) et d'une quantité efficace d'au moins un acide fragrant b) tels que définis selon l'une quelconque des revendications 1 à 6 à une base de produit de soin personnel, où éventuellement lesdits composés a) et b) peuvent être ajoutés sous forme d'une composition de fragrance pré-préparée comprenant lesdits composés a) et b), formant une composition de produit de soin personnel.

9. Méthode selon la revendication 8, dans laquelle la concentration totale du au moins un composé de benzaldéhyde ou de dérivé de benzaldéhyde a) dans la composition de produit de soin personnel va de 0,3 à 0,5%, et la concentration totale du au moins au moins un acide fragrant b) dans la composition de produit de soin personnel va de 0,3 à 0,5%.

10. Méthode selon l'une quelconque des revendications 8 et 9, dans laquelle la composition de produit de soin personnel formée comprend en outre au moins un alcool aromatique ou un dérivé de celui-ci choisi dans le groupe constitué par l'alcool phényléthylique, l'alcool phénylpropylique, le formiate de benzyle, et le formiate de phénéthyle.

11. Méthode selon l'une quelconque des revendications 8 et 9, dans laquelle la composition de produit de soin personnel formée comprend en outre au moins un ingrédient de fragrance antimicrobien tel que défini selon la revendication 4.

12. Méthode selon l'une quelconque des revendications 8 et 9, dans laquelle la composition de produit de soin personnel formée comprend en outre au moins un alcool aromatique ou un dérivé de celui-ci choisi dans le groupe constitué par l'alcool phényléthylique, l'alcool phénylpropylique, le formiate de benzyle, et le formiate de phénéthyle ; et au moins un ingrédient de fragrance antimicrobien tel que défini selon la revendication 4.

13. Méthode selon l'une quelconque des revendications 8 et 9, dans laquelle la composition de produit de soin personnel est un, ou est mise sous forme d'un, produit de soin personnel comprenant les bâtonnets, les roll-on, les sprays, les vaporisateurs à pompe, les aérosols, les pains de savon, les poudres, les solutions, les gels, les crèmes, les baumes et les lotions.
